# EUROPEAN PATENT APPLICATION

(11) **EP 4 495 805 A1**
(43) Date of publication of application: **22.01.2025**
(21) Application number: 23192423.4
(22) Date of filing: 21.08.2023
(51) Int. Cl.: G06F 16/832

(54) **SYSTEM AND METHOD TO GENERATE A SUMMARY TEMPLATE FOR SUMMARIZING STRUCTURED MEDICAL REPORTS**

(30) Priority: 18.07.2023 US 202363527426 P
(71) Applicant: Koninklijke Philips N.V., 5656 AG Eindhoven (NL)
(72) Inventor: NIE, Hongchao, Eindhoven (NL); CHEUNG, Man Ching, Eindhoven (NL)
(74) Representative: Philips Intellectual Property & Standards

(57) **Abstract**

Some embodiments are directed to a method and system to generate a summary template for summarizing structured medical reports. The method may include determining a predictive value for keys and/or key-value pairs in medical reports associated with a given study indication for predicting an associated conclusion, and generating a summary template including selected keys based on the predictive values.

## Description

### FIELD OF THE INVENTION

The presently disclosed subject matter relates to a method to generate a summary template for summarizing structured medical reports, a system to generate a summary template for summarizing structured medical reports, a computer storage medium.

### BACKGROUND OF THE INVENTION

Modern digital health systems aim to consolidate various data sources, forming comprehensive dashboards that enable clinical users to quickly understand a patient's situation. An aspect of this process is the identification and display of Key Data Elements (KDEs) extracted from clinical documents. An example of this process is diagnostic report extraction, particularly diagnostic assessment reports such as echocardiography reports used in ultrasound imaging studies. These reports capture quantitative measurements and qualitative findings, derived from imaging studies, that are integral in affirming or challenging a clinical diagnosis.

Not every measurement or finding holds equal importance to a clinical question at hand. A traditional practice to extract KDEs from echocardiography reports involves manually defining a list of data fields to display on the dashboard. This manual process is labor-intensive and often lacks proper validation. The manual creation of distinct templates to cater to various clinical questions is moreover not scalable, highlighting the need for an improved solution.

Conventional systems utilize natural language processing (NLP) to parse echocardiography reports and find mentions of certain aspects. A known system is described in "Extracting Key Findings Compared In an Echocardiogram Report", by Deyu Sun, Lucas Oliveira, and Kirk Spencer. The known system uses an NLP-based approach that automatically detects which key findings were compared with prior studies and documented in an echocardiography report. To check whether a report contains comparisons with prior studies, words indicating comparisons were searched, such as "compared", "comparison", and so on. Then the sentences related to comparisons were extracted from the raw report and findings that were compared were determined by using concept search. For example, comparisons in LV size, RV systolic functions, etc., may be detected.

The known system may be improved on several fronts. The known system has an inherently limited accuracy due to the reliance on written text and NLP. Indeed, the article points out that accuracy may be improved by incorporating more search patterns to search for in free text. Furthermore, the system is severely limited in its capacity to find aspects, nor is the relative importance of aspects considered. The focus on comparisons is due to the researchers finding this aspect to be important. Adding to or changing of this focus requires manual intervention, in effect, building a new system.

### SUMMARY OF THE INVENTION

The inventors realized that important elements of a report may vary based on the study indication, e.g., the specific clinical question that the referring physician intends to answer. Consequently, a one-size-fits-all approach to creating key data element templates risks either losing important information or including irrelevant data. Furthermore, the manual creation of distinct templates to cater to various clinical questions is not scalable.

Using structured medical reports, rather than free text as in the known system is important advantage. Using a coded system for study indications, key-value pairs, and/or conclusions, at least in part, implies structured findings and measurements at the source, which in turn enables one to spotlight certain measurements and findings in a summary which are more prominent than others in a certain report.

In an embodiment, summary template generation may be automated. Interestingly, this allows the generation of summary templates that are specific for a particular study indication, or even specific for a study indication and cohort. The system may derive the summary templates by analyzing historical structured medical reports with a known conclusion. A structured medical report comprises a list of key-value pairs. For example, the keys may be drawn from a list of known keys, so that the medical report may be parsed automatically. In an embodiment, summarization is only applied to structured parts of the medical report. For example, natural language processing need not be applied to the medical report. In an embodiment, the report does not comprise free text.

A key may be associated with a categorical or numerical value. A categorical may be, in particular, a Boolean, e.g., the key is present or absent. The value may be numerical, e.g., have a numeric value, e.g., an integer or floating point numeral. The multiple report may be analyzed by computing a predictive value of a key and/or of a key-value pair in a medical report for a conclusion associated with the report. Once the predictive value of the various keys are known, a template may be generated based on these values, e.g., keys with a high predictive value are given preference over keys with a low predictive value.

In an embodiment, predictive values are determined by training a machine learning model for the given study indication to predict the report conclusion from the list of key-value pairs for reports associated with the given study indication. The predictive value may be derived from the trained model.

Embodiment may be applied to various type of medical reports, e.g., echocardiography reports.

A system to generate summary templates or to apply summary templates is or includes an electronic system, e.g., an electronic device, e.g., a computer, a processor, a server, etc.

Some embodiments are directed to a method and system to generate a summary template for summarizing structured medical reports. The method may include determining a predictive value for keys and/or key-value pairs in medical reports associated with a given study indication for predicting an associated conclusion, and generating a summary template including selected keys based on the predictive values.

A person skilled in the art will appreciate that the method may be applied to multidimensional image data, e.g., to two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

A further aspect is a method to generate a summary template. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, cloud storage, online software, etc. Preferably, the computer program product comprises (non-)transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium. Another aspect of the presently disclosed subject matter is a method of making the computer program available for downloading.

It will be appreciated by those skilled in the art that two or more of the above-mentioned options, implementations, and/or aspects of the invention may be combined in any way deemed useful.

### BRIEF DESCRIPTION OF DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,
Fig. 1 schematically shows an example of an embodiment of a summary template generation and summary template application system,
Fig. 2a schematically shows an example of an embodiment of a summary template generation system,
Fig. 2b schematically shows an example of an embodiment of a summary template application system,
Fig. 2c schematically shows an example of an embodiment of a conclusion prediction system,
Fig. 3a schematically shows an example of an embodiment of a structured medical report,
Fig. 3b schematically shows an example of an embodiment of a list of predictive values,
Fig. 3c schematically shows an example of an embodiment of a summary template,

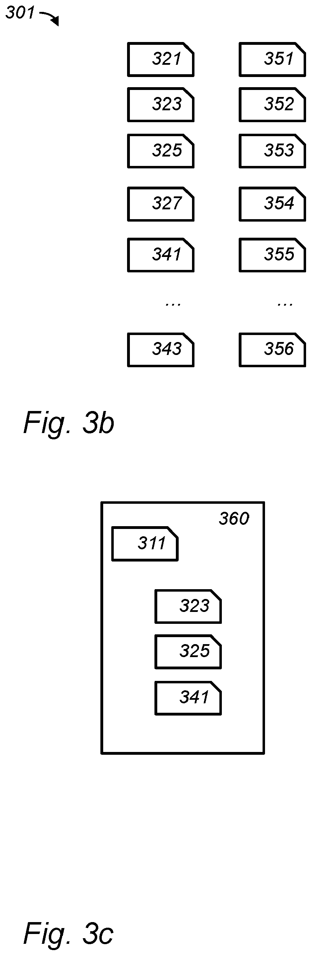

Fig. 3c schematically shows an example of an embodiment of a summary template,

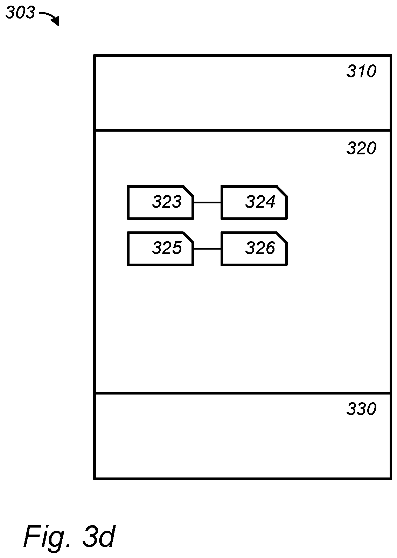

Fig. 3d schematically shows an example of an embodiment of a report summary,

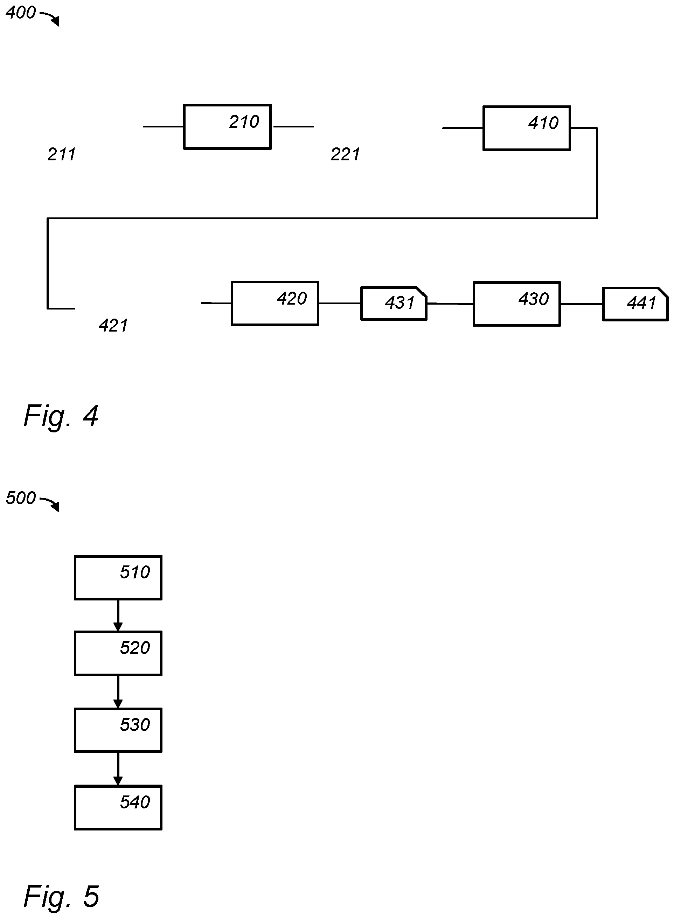

Fig. 4 schematically shows an example of an embodiment of a summary template generation system,
Fig. 5 schematically shows an example of an embodiment of a method to generate a summary template for summarizing structured medical reports,

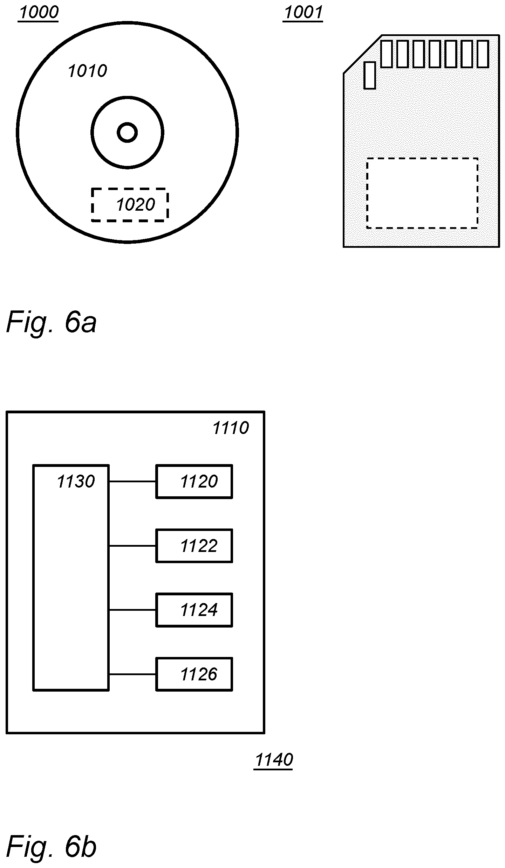

Fig. 6a schematically shows a computer readable medium having a writable part comprising a computer program according to an embodiment,
Fig. 6b schematically shows a representation of a processor system according to an embodiment.

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

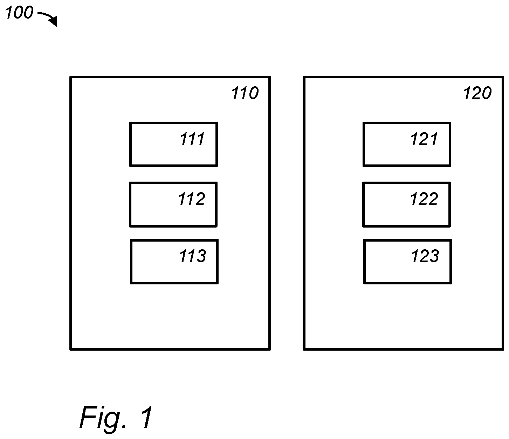

Further, the subject matter that is presently disclosed is not limited to the embodiments only, but also includes every other combination of features described herein or recited in mutually different dependent claims. Fig. 1 schematically shows an example of an embodiment of a summary template generation and summary template application system 100. System 100 comprises a summary template generation system 110 and a summary template application system 120. Summary template generation system 110 is configured to generate a summary template for summarizing structured medical reports. Given multiple structured medical reports for a given study indication, system 110 generates a summary template. The summary template may be used for automatic summarization of a medical report of that type. A structured medical report comprises a list of key-value pairs in a computer-parsable data structure. The data structure can be read and interpreted by systems 110 and 120, and which systems are configured to break down the data into its constituent parts, e.g., keys and associated values, according to a data format of the data structure. For example, the structured medical report may comprise key-value pairs structured according to one or more of JSON, XML, YAML, CSV, Protocol Buffers, and Apache Avro.

Summary template application system 120 is configured to apply a summary template generated by system 110. For example, system 120 may receive a structured medical report, obtain a corresponding summary template, and apply the template to the report, obtaining a summary of the report according to the template. System 120 may parse the structured medical report according to the same data format as is used by system 110.

For example, the system 100 may be used in medical setting, wherein medical practitioners need to have a quick understanding of the salient points in a medical report. By providing a summary first, or in addition to a report, medical practitioner can see at a glance which key-values in the report are likely to be determinative for a conclusion based on the report, or for a subsequent medical treatment or the like.

Summary template generation system 110 may comprise a processor system 111, a storage 112, and a communication interface 113. Summary template application system 120 may comprise a processor system 121, a storage 122, and a communication interface 123.

In the various embodiments of communication interfaces 113 and/or 123, the communication interfaces may be selected from various alternatives. For example, the interface may be a network interface to a local or wide area network, e.g., the Internet, a storage interface to an internal or external data storage, an application interface (API), etc.

Storage 112, and 122 may be, e.g., electronic storage, magnetic storage, etc. The storage may comprise local storage, e.g., a local hard drive or electronic memory. Storage 112, and 122 may comprise non-local storage, e.g., cloud storage. In the latter case, storage 112, and 122 may comprise a storage interface to the non-local storage. Storage may comprise multiple discrete sub-storages together making up storage 112, and 122.

Storage 112 and/or 122 may be transitory or non-transitory storage. For example, storage 112 and/or 122 may store data in the presence of power such as a volatile memory device, e.g., a Random Access Memory (RAM). For example, storage 112 and/or 122 may store data in the presence of power as well as outside the presence of power such as a non-volatile memory device, e.g., Flash memory. Storage may comprise a volatile writable part, say a RAM, a non-volatile writable part, e.g., Flash. Storage may comprise a non-volatile non-writable part, e.g., ROM.

System 110 and system 120 may have access to a storage, e.g., a database, e.g., for storing medical reports, summaries, templates, models, and the like.

The systems 110 and 120 may communicate internally, with each other, with other systems, external storage, input devices, output devices, and/or one or more sensors over a computer network. The computer network may be an internet, an intranet, a LAN, a WLAN, etc. The computer network may be the Internet. The systems 110 and 120 may comprise a connection interface which is arranged to communicate within system 100 or outside of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. The communication interface 113 may be used to send or receive digital data, e.g., medical reports, and templates. The communication interface 123 may be used to send or receive digital data, e.g., medical reports, templates, and summaries.

Summary template generation system 110 and summary template application system 120 may have a user interface, which may include well-known elements such as one or more buttons, a keyboard, display, touch screen, etc. The user interface may be arranged for accommodating user interaction for creating a new template and/or summary.

The execution of systems 110 and 120 may be implemented in a processor system. The systems 110 and 120 may comprise functional units to implement aspects of embodiments. The functional units may be part of the processor system. For example, functional units shown herein may be wholly or partially implemented in computer instructions that are stored in a storage of the system and executable by the processor system. System 110 and/or 120 may be implemented in a device, e.g., in a computer, e.g., a server, a workstation, etc.

The processor system may comprise one or more processor circuits, e.g., microprocessors, CPUs, GPUs, etc. Systems 110 and 120 may comprise multiple processors. A processor circuit may be implemented in a distributed fashion, e.g., as multiple sub-processor circuits. For example, systems 110 and 120 may use cloud computing.

Typically, the summary template generation system 110, summary template application system 120 each comprise one or more microprocessors which executes appropriate software stored at the system; for example, that software may have been downloaded and/or stored in a corresponding memory, e.g., a volatile memory such as RAM or a non-volatile memory such as Flash.

Instead of using software to implement a function, the systems 110 and 120 may, in whole or in part, be implemented in programmable logic, e.g., as field-programmable gate array (FPGA). The systems may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, systems 110 and 120 may comprise circuits, e.g., for cryptographic processing, and/or arithmetic processing.

In hybrid embodiments, functional units are implemented partially in hardware, e.g., as coprocessors, and partially in software stored and executed on the system.

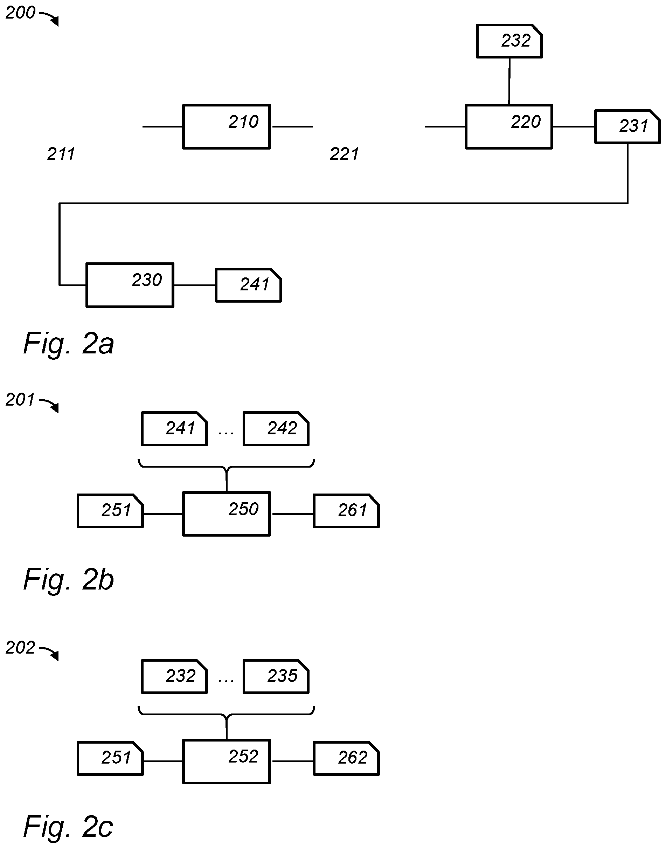

Fig. 2a schematically shows an example of an embodiment of a summary template generation system 200. System 200 is configured to generate a summary template. Summary template may later be used for summarizing structured medical reports.

System 200 is configured to obtain multiple structured medical reports 211. For example, the medical reports may be reports of a medical imaging procedure. For example, the medical reports may comprise echocardiography reports, e.g., a report of an echocardiographic examination of a patient's heart.

The medical reports are arranged in a data format suitable for automated parsing. A medical report may have three parts: one or more study indications, a list of key-value pairs, and a conclusion. During training typically all of the medical reports have a conclusion. During application of a summary template, a report may or may not have a conclusion, or may not yet have a conclusion.

The study indication, also referred to as the clinical indication, or the clinical question, generally refers to the reason or rationale for performing a particular study, test, or procedure. For instance, in the context of diagnostic imaging, the study indication would be the specific medical reason why a physician has ordered an imaging study like an X-ray, MRI, or CT scan. The study indication may represent the reason for performing a particular study or test.

The study indication may refer to the need to monitor an existing condition, or to rule out possible conditions based on the patient's clinical presentation. For example, if a patient has a persistent cough and shortness of breath, the study indication for a chest X-ray might be "evaluation of potential pneumonia", possibly further coded in a computer parsable format.

Preferably, the study indication is coded according to a standard coding for study indications or clinical indications. The study indication may follow a classification system providing a specific code for study indications, e.g., the study indications expected to be covered by the users of the summary templates generated by system 200.

A key-value pair may indicate the presence or absence of a medical feature. For example, the value may be of Boolean type. For example, the following are key-value pairs: LV-dilatation: True; Hypertrophic cardiomyopathy: Yes; Aortic stenosis: No. A key value pair may also indicate a measurement. For example, a key may be a measurement type, and the value a calculated or measured quantity. For example, Key value pairs may be: LV diastolic diameter measurement (mm): 5; LV size finding: normal; RA size finding: 0,7 At least part of the key-value pairs may represent a medical measurement taken on a human body. The measurement may be taken using medical imaging. At least part of the key-value pairs may represent the presence or absence of a finding code.

The key-value pairs may be encoded according to a standard as well. A suitable standard may be imposed by the workstations used by the medical practitioners. For example, IntelliSpace Cardiovascular ISCV may be used.

The conclusion may indicate at least a presence or absence of a specific medical condition, e.g., an anomaly. Preferably, the conclusion corresponds to the study indication. For example, a conclusion may comprise a value answering the clinical question comprised in or associated with the study indication. For example, the value may be a Boolean value indicating the presence or absence of the study indication, or a yes/no answer. For example, for a study indication "evaluation of potential pneumonia", the conclusion may comprise a Boolean value indicating whether or not pneumonia was established in the examination, e.g., the medical imaging examination.

A conclusion may comprise a so-called finding code. Finding codes in the medical field typically refer to the specific codes used in a standardized medical coding systems to record and communicate medical findings, e.g., diagnoses, symptoms, abnormal findings, etc. These codes may be used for a variety of purposes such as treatment planning, medical records management, epidemiological research, etc. The finding code may be used for classifying and coding diagnoses, and abnormal findings, e.g., following an examination, e.g., a medical imaging examination.

The finding codes may follow a medical coding system that provides a standardized way to categorize various medical conditions. The standard may be a local standard, e.g., as used in a particular hospital, or range of hospitals, or an internal standard within the reporting software. The standard may be an external standard, e.g., the International Classification of Diseases (ICD). The conclusion can be coded with a finding code that is not an industry standard. Any self-consistent coding scheme used within the product will work.

The conclusion may reference one or more keys of the list of key-value pairs. For example, a medical practitioner may refer to one or more particular keys, and possibly the corresponding value, to justify his/her conclusion. It is not necessary for a conclusion to refer to the keys, but if this is done, the keys in the conclusion may be used as a signal to assign a predictive value to keys.

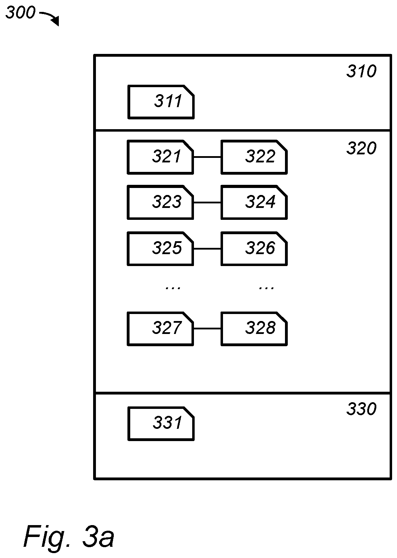

Fig. 3a schematically shows an example of an embodiment of a structured medical report 300. Report 300 comprises a study-indicator segment 310, a key-value segment 320, and a conclusion segment 330.

Study-indicator segment 310 comprises at least a study indicator 311. Segment 310 may comprise further information, e.g., personal information regarding the patient that is not predictive of the person's medical condition, e.g., administrative information of the patient, such as a name or number.

Key-value segment 320 comprises a list of key-value pairs. Shown are keys: 321, 323, 325, and 327. Shown are the corresponding keys: 322, 324, 326, and 328.

Conclusion segment 330 comprises a conclusion 331. For example, conclusion 331 comprises a Boolean value answering study indication 311.

The particular way to organize report 300 shown in Fig. 3a is an example, and other ways of organizing the information may be used. For example, conclusion segment 330 may be included above segment 320. For example, no segments may be used, e.g., part of the information in segment 310 may be included in key-value pairs as well. For example, a patient name, number, etc., may be part of a key-value pair list.

Interestingly, a study indication may be missing in a structured report as an independent element, but a study indication may nevertheless be derived from the report. For example, a study indication may be derived from the conclusion. For example, report 300 may miss study indication 311 and/or segment 310, but does include conclusion 331. For example, in an embodiment multiple sets of conclusions, e.g., conclusions codes, are mapped to multiple respective study indications. Derived study indications may be filled-in report 300, or they may be derived on the fly when needed. For example, the conclusions 'no pneumonia' and 'pneumonia' may both be mapped to the study indication `evaluation of potential pneumonia'.

Returning to Fig. 2a.

System 200 generates a summary template for a given study indication of the multiple study indications. System 200 comprises a medical reports selector 210. Selector 210 selects from the multiple structured medical reports 211 those that have a given study indication. The study indication may be given by a user of system 200 who desires a summary template for that particular study indication. The study indication may also be automatically generated. For example, a summary template may be generated for all study indications, or for all study indications for which a minimum number of reports are available.

In addition to the study indication, selector 210 may further restrict the reports. For example, only medical reports that are associated with a specific cohort may be selected. Accordingly, system 200 will generate a summary template for the given study indication and the given specific cohort. For example, the cohort may be based on age, e.g., adults over 65, the cohort may be based on gender, e.g., only male or female patients, lifestyle specific, e.g., only smokers, etc. Information indicating the cohort may be included in a report, e.g., in segment 310.

System 200, e.g., selector 210, is configured to determine multiple structured medical reports 221, restricted according to study indication, and possibly further information. Given the restricted list of reports 221, a predictive value is determined for the keys and/or key-value pairs in medical reports 221 for predicting the associated conclusion in medical reports 221.

System 200 may comprise a predictive value determiner 220 configured to determine predictive values 231 for the keys that indicate how useful a key is in predicting the conclusion of the report. Typically, a predictive value is computed for each key that occurs at least once in reports 221, though in an embodiment underrepresented keys may be left out. For example, keys that occur in fewer than a predetermined number or fraction of the reports 221 may be left out. No predictive value may be assigned to the left out keys. Some rule may be followed to deal with such keys in summarization, for example, should such a key occur in a report, it could always be left out, or always included, or yet other options.

One way to obtain predictive values is to train a machine-learned prediction model 232. Model 232 may be configured to take as input the key-value pairs in a report, e.g., a report from multiple reports 221 and to predict the conclusion of the report in the output. Predictive value determiner 220 may be configured to train machine learning model 232 for the given study indication to predict the report conclusion from the list of key-value pairs for reports associated with the given study indication.

Having trained model 232 the predictive values may be derived from the trained model. This approach, training a model to get predictive values, is not necessary, but it provides validated and well quantified predictive values, which have a high explanatory value as well.

In an embodiment, the conclusion comprises a Boolean value. Accordingly, predicting the conclusion may be modelled as a classification problem. Optionally, before training the reports may be preprocessed. For example, the key-value pairs in each report may be represented as a vector in a multidimensional space. Each dimension corresponds to a key in a list of multiple keys. The list of keys may be built from the entire set of structured reports. The value in each dimension may be the corresponding value of the key, possibly normalized to a standardized scale, e.g., a floating point value between 0 and 1 inclusive. Boolean keys may be presented as 0 or 1 values in the vector. Converting to a vector may not be necessary if the data format of the reports is sufficiently suitable for machine learning. Alternatively, the vector representation may be done on the fly when a particular report is used.

The vectors are used to train a machine learning model. Algorithms such as Naive Bayes, Logistic Regression, or Support Vector Machines may be used for this classification problem. If the amount of data permits it, a neural network-based approach, such as a Transformer or a LSTM model, may be used.

Having the trained model, it may be used to determine the predictive value, or weight, for each key. That is which keys are important because they are the most predictive of a report's conclusion. For example, the predictive value may be derived from the weights, e.g., coefficients, assigned by the model to each key. In a linear model like Logistic Regression, a higher absolute weight means the word is more predictive of the outcome. If the weight is positive, it predicts one class; if it's negative, it predicts the other. For example, the absolute value of the weight may be scaled to a range, say 0-1, as the predictive value. Likewise, if the model includes an attention mechanism, such as a transformer model, then the attention weights may be used as an indication of the importance of each key for the output.

In an embodiment, the model comprises one or more decision trees. The predictive value for a key may be derived at least from the number of times the key occurs in the one or more decision trees.

Instead of deriving predictive values from model coefficient directly, a model explanation algorithm may be used. For example, Partial Dependence Plots (PDP) may be used to attach an importance to a key given the trained model. See, e.g., Greenwell, Brandon M., Bradley C. Boehmke, and Andrew J. McCarthy. "A simple and effective model-based variable importance measure." arXiv preprint arXiv: 1805.04755 (2018). For example, Permutation Feature Importance may be used. For example, an embodiment may comprise distorting the values of a key and then measuring the decrease in the model's performance. If distorting a particular feature leads to a large decrease in performance, that feature get a higher predictive value. It may not be necessary to retrain the model. Instead, a key's predictive value may be derived by evaluating the model for multiple reports with or without a correct or distorted value for a particular key. A predictive value can be computed for a key that indicates how likely the model's outcome is to change because of the distorted value, e.g., on the average.

Fig. 3b schematically shows an example of an embodiment of a list 301 of predictive values. Shown in Fig. 3b are a list keys: keys 321, 323, 325, 327, 341, and 343, and the corresponding predictive values: 351, 352, 353, 354, 355, 356. Assuming that report 300 is included in training data 221, we see that all keys in report 300 are indeed included in list 301. However, more keys are included in list 301, as shown, keys 341 and 343, because multiple reports have been taken into account to create list 301. Not all keys are used in all reports. For example, keys 341, and 343 are used in some reports, but not in report 300.

Returning to Fig. 2a.

Instead of training different models for different study indications, or for different combinations of study indication and other information, e.g., cohorts, it is also possible to train a single larger machine learning model that also includes the study indication and optional other information in its input, in addition to the key-value pairs. The single model is trained to predict the report conclusion from the list of key-value pairs and the study indication, and optional other information.

Predictive values may now be derived for the given study indication by determining the predictive values from the single machine learning model, wherein the single model is used for multiple study indications. For example, to compute predictive values for a particular study indication, the study indication input may temporarily be fixed to the particular study indication, after which the algorithms discussed herein may be used to compute predictive values.

It is not necessary to train a model to obtain predictive values. For example, in an embodiment, the report's conclusion references one or more keys of the list of key-value pairs. For example, a medical practitioner may refer to a particular key to substantiate a diagnosis reached in the conclusion. For a given key, system 200, e.g., unit 210 may count the number of medical reports that reference the given key in a conclusion, and the number of medical reports for the given study indication that reference the given key in the conclusion. The predictive value may be derived from said counts.

For example, a first fraction of reports having the study indication and referencing a key may be computed; a second fraction of reports across all study indications that reference the key may be computed. The predictive value may be the first fraction divided by the second fraction, or first fraction divided by the log of the second fraction.

In this case no explicit model is trained, yet predictive values, that indicate the importance for a conclusion are nonetheless computed.

System 200 comprises a template generator 230 configured to generate a summary template 241. Template generator 230 is configured to select multiple keys that occur in at least one of the lists of key-value pairs of medical reports associated with the given study indication, e.g., from list 231. Template generator 230 bases the selection on the determined predictive value of the keys. In an embodiment, the values are also used, e.g., the selection may be based on key-value pairs, see elsewhere herein. Having selected the keys, a summary template is generated specific for the given study indication. The summary template comprises the selected multiple keys. With such a summary template a summary for a report can be created by extracting from the structured medical reports the selected keys that are listed in the summary template.

For example, template generator 230 may be configured to select the keys, e.g., from list 231, that have a predictive value over a threshold value. The threshold value may be predetermined, e.g., experimentally. Alternatively, a set of keys may be selected with highest predictive values, wherein the set has a determined number of keys. A combination may also be used, all keys with a predictive value over a threshold, but with a minim number of keys, e.g., including additional keys if the number of selected keys is too small.

Template generator 230 may be programmed to exclude certain keys from the summary template. For example, a key which is equivalent with the conclusion, and thus highly correlated therewith, may nevertheless be excluded from the summary. For example, a key which indicates that a person is a known heart patient will correlate highly with the conclusion that the examination indicates heart problems. For example, generator 230 may be configured to only select keys from a predetermined set of keys, e.g., only keys that relate to the examination, but not keys that relate to medical history. For example, keys relating to medically unimportant details, e.g., personal names, may also be left out.

In an embodiment, system 200, e.g., unit 210, also trains a general machine learning model to predict the conclusion, e.g., the presence or absence of the specific medical condition from the list of key-value pairs independent of the study indication. From the general model, general predictive values may be derived, in a like manner as for the study indication specific model.

Unit 230 may select one or more additional keys for the summary template based on the determined general predictive value. Keys with a generally speaking high predictive value may thus be included even if they are not particularly predictive for the given study indication. For example, it may happen that some problem could be identified or suspected from the full report, but which problem is unrelated to the study indication. Because of that, such keys may not be included in the summary template. Using general predictive value allows adding in one or more keys that are generally predictive of conclusions.

Including keys based on general predictive values is particularly useful when values are also included in predive value determination. The result of this being that general keys may not normally be included in the summary template for a specific study indication, but are included if they have a particular predive value.

In an embodiment, unit 230 applies a hierarchical clustering algorithm to the study indications. The clustering algorithm may be used to establish a hierarchical relationship between study indications. Suppose a first study indication and a second study indication that are hierarchically related according to the hierarchical clustering algorithm. Also assume that the first study indication is closer to the root of the hierarchical clustering than the second key. In this case, a key selected for the first study indication may automatically be selected for the second study indication as well.

Automatic hierarchical grouping of features may be used in unsupervised learning to group similar instances on the basis of their key-value pairs. In an embodiment, the clustering is based on a distance function between study indications. The distance between two study indications may be a function that is inversely proportional to the amount of overlap in key-value pairs in reports corresponding to the study indications.

In an embodiment, the multiple keys are selected using a recursive feature elimination algorithm that iteratively considers a smaller set of keys. The Recursive Feature Elimination (RFE) algorithm is a feature selection method that fits a model and removes the weakest feature (or features) until a specified number of features is reached. After one or a few features have been eliminated, the predictive values for the remaining keys may be recomputed, and new features may be selected for removal.

In an embodiment, a medical practitioner may provide feedback on a summary template. System 200 may be configured to receive said feedback and to include and/or exclude a key from the summary template based on the feedback.

Fig. 3c schematically shows an example of an embodiment of a summary template 360. In this example, summary template 360 comprises the study indication 311 for which it is intended, and keys that are to be extracted. Shown are keys 323, 325 and 341.

Fig. 2b schematically shows an example of an embodiment of a summary template application system 201. System 201 comprises a summary template applicator 250. Applicator 250 has access to multiple summary templates; shown are summary templates 241 and 242. For example, the multiple summary templates may be a summary template such as template 360. Applicator 250 receives as input a structured medical report 251. Structured medical report 251 comprises a list of key-value pairs. Medical report 251 is associated with one of multiple study indications. Accordingly, applicator 250 selects the summary template for the associated study indication. The keys that are both in report 251 and the summary template are included in the summary.

The summary may be shown on a display to the user, e.g., as a pop-up, or the like.

Fig. 3d schematically shows an example of an embodiment of a report summary 303. In this example, summary template 360 is applied to report 300. The summary may include further elements, e.g., as determined by the user, e.g., part or all of the study indication segment, and the conclusion segment may be included.

Fig. 2c schematically shows an example of an embodiment of a conclusion prediction system 202. System 202 comprises a prediction model applicator 252 to predict a conclusion from a list of key-value pairs. Applicator 252 has access to multiple machine-learned prediction model; shown are machine-learned prediction models 232 and 235. Applicator 252 receives as input a structured medical report 251. Structured medical report 251 comprises a list of key-value pairs. Medical report 251 is associated with one of multiple study indications. Accordingly, applicator 252 selects the prediction model for the associated study indication. The model is applied to the key-pairs in report 251.

The predicted conclusion may be reported to a user. The predicted conclusion may be shown on a display to the user, e.g., as a pop-up, or the like. For example, a user may review and/or correct the conclusion before including the conclusion in report 251. In an embodiment, the predicted conclusion is initially not shown to the user, but a user writing a conclusion is warned if the written conclusion deviates from the predicted conclusion.

An embodiment may comprise a report writing unit configured to receive input from a medical practitioner, e.g., through a keyboard or voice assistant. The report writing unit may comprise a quality verification, e.g., which activates as soon as the practitioner proceeds to finalize the report and/or the practitioner may trigger the quality verification him/herself. The quality verification may include checks such as spell check, or checks for completeness, e.g., a missing conclusion, but may also compare the conclusion with a predicted conclusion.

In an embodiment, conclusion prediction may run in the background updating its prediction as more data is entered into the report. A prediction made by the conclusion prediction may be presented upon request or if the conclusion prediction surpasses a confidence threshold. In an embodiment, a log is kept of clinical feedback. Clinical feedback may include feedback on a summary, e.g., a request to include or exclude a key data element. Clinical feedback may include if a practitioner accepts or rejects a predicted conclusion. The latter feedback may be used to further train the prediction model, e.g., the model may be fine-tuned.

The report writing unit may allow entry of a report in natural language and convert the entry into a structured report. The report writing unit may support directly entering structured data, e.g., by offering menus arranged for the selection of keys.

Fig. 4 schematically shows an example of an embodiment of a summary template generation system 400. System 400 is similar to system 200 but preprocesses the key-value pairs before generating predictive values.

System 400 receives a set of structured medical reports 211, from which those reports 221 are selected that relate to a particular study indication, e.g., by a selector 210. System 400 comprises a range discretization unit 410. Unit 410 converts a key-value pair, in which the value is not Boolean, but may have multiple values, e.g., integers and floating points values, into multiple new key-value pairs, in which the new values are Boolean. This is done by portioning the original range of the value in the original key-value pair, into two or more ranges. The new values in the new key-value pairs indicate if the original value lies in the corresponding new range.

For example, for a given key, unit 410 may perform the following:
1. determine the range of the corresponding value from data 221
2. partitioning the range into at least two new ranges
3. for each key-value pair with this key, create a set of new Boolean key-value pairs corresponding to the partitioned ranges that indicate if the original value lies in a corresponding range. The original key-value pair may be discarded or kept.

Instead of Boolean values, a categorized value may be used instead.

To determine the range, several approaches are possible. For example, the values in the range from phase 1, may be modelled as Normal distributed, the ranges may correspond to a number of standard deviations from the mean, e.g., between 0-1 standard deviations removed, between 1-2 standard deviations removed, etc.

Other approaches are possible. For example, a correlation with the conclusion and the key-value pairs may be used to determine the ranges. For example, one may use decision trees to find ranges that maximize the separation between the classes. Once ranges are determined, the decision tree may be used to determine predictive values, or an alternative model may be trained on them.

Key-value pairs in data 221 is converted into multiple discretized structured medical reports 421 having Boolean key-value pairs, e.g., by binning the data into ranges and associating a Boolean variable with reach range. A predictive value determiner 420 may now be used. This determiner may work similarly to determiner 220. The outcome is predictive values 431 for the binned key-value pairs. A summary template may be generated by a template generator 430 to generate a summary template 441. The summary template not only contains keys, but also ranges associated with the keys. To apply the template, a key-value pair is included in a summary if the key is in the summary template and the corresponding value is in the range indicated by the summary template. Many values in a medical report are not particularly informative, unless they have an aberrant value. Using system 400 such key-value pairs are selected by a summary generator.

System 400 may be combined with system 200. For example, a first set of keys may be determined by system 200. These keys are always included in a summary if they are present regardless of their values. A second set of keys may be determined by system 400. These keys are only included in a summary if they are present in a report and their value lies in the correct range. Interestingly, the second set can be chosen relatively large, if the ranges are relatively small.

Fig. 5 schematically shows an example of an embodiment of a method 500 to generate a summary template for summarizing structured medical reports. Method 500 may be computer implemented. Method 500 may comprise
- obtaining (510) multiple structured medical reports, the structured medical reports comprising a list of key-value pairs, each medical report being associated with one of multiple study indications, each medical report being associated with a conclusion indicating at least a presence or absence of a specific medical condition,
- for a given study indication of the multiple study indications
   - determining (520) a predictive value for keys and/or key-value pairs in medical reports associated with the given study indication for predicting the associated conclusion,
- selecting (530) multiple keys that occur in at least one of the lists of key-value pairs of medical reports associated with the given study indication based on the determined predictive value of the keys and/or key-value pairs,
- generating (540) a summary template for the given study indication, the summary template comprising the selected multiple keys, the summary template allowing the extracting from structured medical reports of the selected keys for automatic summarization of the medical report.

The method may be applied to a large number of reports, e.g., at least 100, at least 1000. The model may comprise training a model. The trained model may have a large number of coefficients, e.g., at least 100, at least 1000, or at least 10000. The number of study indications may be at least 5, at least 10, at least 100, or at least 1000. The number of keys may be at least 100, at least 1000, or at least 10000.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 500. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

Fig. 6a shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of a method to generate a summary template, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform an embodiment of said method to generate a summary template.

Fig. 6b shows in a schematic representation of a processor system 1140 according to an embodiment of a summary template generation system. The processor system comprises one or more integrated circuits 1110. The architecture of the one or more integrated circuits 1110 is schematically shown in Fig. 6b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the summary template generation system or device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While system 1140 is shown as including one of each described component, the various components may be duplicated in various embodiments. For example, the processing unit 1120 may include multiple microprocessors that are configured to independently execute the methods described herein or are configured to perform steps or subroutines of the methods described herein such that the multiple processors cooperate to achieve the functionality described herein. Further, where the system 1140 is implemented in a cloud computing system, the various hardware components may belong to separate physical systems. For example, processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

### Reference signs list

The following list of references and abbreviations corresponds to Figs. 1-4, and is provided for facilitating the interpretation of the drawings and shall not be construed as limiting the claims.
- 100: a summary template generation and summary template application system
- 110: a summary template generation system
- 120: a summary template application system
- 111, 121: a processor system
- 112, 122: storage
- 113, 123: communication interface
- 200: a summary template generation system
- 201: summary template application system
- 202: a conclusion prediction system
- 210: medical reports selector
- 211: multiple structured medical reports
- 220: predictive value determiner
- 221: multiple structured medical reports
- 230: template generator
- 231: predictive values
- 232: machine-learned prediction model
- 235: machine-learned prediction model
- 241: a summary template
- 242: a summary template
- 250: a summary template applicator
- 251: a structured medical report
- 252: a prediction model applicator
- 261: a summary
- 262: a prediction
- 300: a structured medical report
- 301: a predictive values list
- 303: a summary
- 310: a study-indicator segment
- 311: a study indicator
- 320: a key-value segment
- 321,323: a key
- 325, 327 322, 324: a value
- 326, 328 330: a conclusion segment
- 331: a conclusion
- 341,343: a key
- 351-356: a predictive value
- 360: a summary template
- 400: a summary template generation system
- 410: a range discretization unit
- 420: predictive value determiner
- 421: multiple discretized structured medical reports
- 430: template generator
- 431: predictive values
- 441: a summary template
- 1000, 1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

## Claims

1. A computer implemented method (500) of generating a summary template for summarizing structured medical reports, the method comprising:
- obtaining (510) a plurality of structured medical reports, the structured medical reports comprising a list of key-value pairs, each medical report of the plurality of structured medical reports being associated with (i) one of multiple study indications, and (ii) a conclusion indicating at least a presence or absence of a specific medical condition,
- for a given study indication of the multiple study indications
- determining (520) a predictive value for keys and/or key-value pairs in medical reports associated with the given study indication for predicting the associated conclusion,
- selecting (530) multiple keys that occur in at least one of the lists of key-value pairs of medical reports associated with the given study indication based on the determined predictive value of the keys and/or key-value pairs,
- generating (540) a summary template for the given study indication, the summary template comprising the selected multiple keys, the summary template allowing the extracting from structured medical reports of the selected keys for automatic summarization of the medical report.

2. The method as claimed in claim 1, comprising:
- training a machine learning model for the given study indication to predict the conclusion of the medical report from the list of key-value pairs for reports associated with the given study indication and deriving the predictive values from the trained model.

3. The method as in any of the preceding claims, comprising:
- training a machine learning model to predict the presence or absence of the specific medical condition from the list of key-value pairs independent of the study indication and deriving a general predictive value therefrom,
- selecting one or more general keys based on the determined general predictive value, and
- including the selected general keys in the summary template for the given study indication.

4. The method as in any of the preceding claims, wherein
- at least part of the key-value pairs represent a medical measurement previously taken on a human body, and/or
- at least part of the key-value pairs represents the presence or absence of a finding code.

5. The method as in any of the preceding claims, wherein
- keys are selected having a predictive value over a threshold value, and/or
- a set of keys is selected with highest predictive values, wherein the set has a determined number of keys.

6. The method as in any of the preceding claims, wherein the conclusion references one or more keys of the list of key-value pairs, the method comprising:
- for a given key, counting the number of medical reports that reference the given key in a conclusion, and the number of medical reports for the given study indication that reference the given key in the conclusion,
- deriving a predictive value from said counts.

7. The method as in any of the preceding claims, wherein a hierarchical clustering algorithm is applied to the study indications, given a first study indication and a second study indication that are hierarchically related according to the hierarchical clustering algorithm with the first study indication being closer to the root of the hierarchical clustering than the second key, a key selected for the first study indication being also selected for the second study indication.

8. A method as in any of the preceding claims, wherein the medical reports are associated with a specific cohort, the method generating a summary template for the given study indication and for a given specific cohort.

9. The method as in any of the preceding claims, comprising:
- determining a predictive value for a key and multiple ranges of the corresponding value,
- selecting a key and a range of the key based on the determined predictive value of the key and the range,
- the summary template comprising the selected key and range, the summary template allowing the extracting from structured medical reports of the selected key if the value of the key is in the range.

10. The method as in any of the preceding claims, wherein
- selecting multiple keys comprises a recursive feature elimination algorithm that iteratively considers a smaller set of keys, and/or
- the model comprises one or more decision trees, wherein the predictive value for a key is derived at least from the number of times the key occurs in the one or more decision trees.

11. The method as in any of the preceding claims, comprising:
- training a machine-learning model to predict the report conclusion from the list of key-value pairs and the study indication, the model receiving as input at least the list of key-value pairs and the study indication,
- for the given study indication determining the predictive values from the single machine learning model, wherein the single model is used for multiple study indications.

12. A computer implemented method of summarizing a structured medical report, the method comprising
- obtaining a structured medical report, the structured medical report comprising a list of key-value pairs, the medical report being associated with one of multiple study indications,
- retrieving a summary template for one study indication, the summary template comprising multiple selected keys, the summary template being obtained from a method according to any of claims 1-11, and
- extracting from the structured medical reports the selected keys and their corresponding values and including them in a summary of the medical report, and causing the summary to be displayed to a user.

13. The method as in Claim 12, comprising predicting a conclusion for the structured medical report by applying the machine learning model to the list of key-value pairs in the medical report and the study indication, wherein
- the predicted conclusion is reported to a user, and/or
- a user writing a conclusion is warned if the written conclusion deviates from the predicted conclusion.

14. A system for generating a summary template for summarizing structured medical reports, the system comprising one or more processors and one or more storage devices storing instructions that, when executed by the one or more processors, cause the one or more processors to perform operations according to the method of any of the preceding claims.

15. A computer storage medium encoded with instructions that, when executed by one or more processors, cause the one or more processors to perform operations according to any of Claims 1-13.
